(19) Europäisches Patentamt
European Patent Office
Office européen des brevets

(11) Publication number: 0 317 175 B1

(12) **EUROPEAN PATENT SPECIFICATION**

(45) Date of publication of patent specification: **22.09.93**

(51) Int. Cl.5: **C07D 239/26**, C09K 19/34

(21) Application number: **88310581.9**

(22) Date of filing: **10.11.88**

The file contains technical information submitted after the application was filed and not included in this specification

(54) **Pyrimidine derivatives.**

(30) Priority: **17.11.87 JP 289690/87**

(43) Date of publication of application:
**24.05.89 Bulletin 89/21**

(45) Publication of the grant of the patent:
**22.09.93 Bulletin 93/38**

(84) Designated Contracting States:
**CH DE GB LI**

(56) References cited:
**WO-A-86/04081**
**WO-A-88/02018**
**GB-A- 2 092 169**

(73) Proprietor: **SEIKO EPSON CORPORATION**
**4-1, Nishishinjuku 2-chome**
**Shinjuku-ku Tokyo-to(JP)**

(72) Inventor: **Yamada, Shuhei**
**c/o Seiko Epson Corporation 3-5 Owa**
**3-chome**
**Suwa-shi Nagano-ken(JP)**

(74) Representative: **Miller, Joseph et al**
**J. MILLER & CO. 34 Bedford Row, Holborn**
**London WC1R 4JH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

This invention relates to pyrimidine derivatives useful as electro-optical display materials.

Generally, liquid crystal display devices use the electro-optical effect possessed by liquid crystal materials. In particular, liquid crystal display devices using the nematic liquid crystal phase are of one of the following types: twisted nematic; voltage controlled bi-refringence; guest-host; and dynamic scattering. Recently, as an improvement of the twisted nematic type, a super-twisted nematic type in which the conventional twist angle of 90° is increased to an angle of 180° to 250°, has been put into practical effect. Further, a super-twisted controlled bi-refringence type using the transition between the stable condition in which the twist angle thereof is 270°, has been proposed although it has not yet been put into practical effect.

Liquid crystal display devices have some advantageous features, for example, they can be driven by relatively low voltage and have relatively low power consumption, and may be made small and flat, and there is no eye strain because of their passive structure even if they are used over long periods of time. Therefore, liquid crystal display devices have been widely applied in the fields of watches, electronic calculators, dash boards of cars, audio mechanisms and so on. Recently, liquid crystal display devices have been applied to personal computers and word processor displays and pocket colour televisions in place of the conventional cathode ray tube.

The liquid crystal material used in liquid crystal display devices depends on the field of application, because different fields require liquid crystal materials with different properties. For example, the twisted nematic type of liquid crystal material, which is used widely at present, has the following properties:

1. colourless, and stability in view of thermal, optical, electrical and chemical influences;
2. wide range of temperature suitable for practical use;
3. high speed of electro-optical response;
4. relatively low driving voltage;
5. sharpness of the rise time of the voltage-light transmittance, and relatively low temperature dependence;
6. wide visual range.

Among the properties particularly important for practical use are relatively low driving voltage and relatively wide temperature range. In the case of the twisted nematic type of liquid crystal material, the following relationship can be found between threshold voltage $V_{th}$ and the dielectric anisotrophy $\Delta \Sigma$.

$$V_{th} = k \sqrt{\frac{K_{11} + 1/4\,(K_{33} - 2K_{22})}{\Delta \varepsilon}}$$

wherein, each of $K_{11}$, $K_{22}$ and $K_{33}$ is an elastic constant relative to spray, twist and bend, respectively, $\Delta \Sigma$ is the dielectric anisotropy, and k is a proportional constant. Therefore, in order to increase the threshold voltage, a positive and large value of $\Delta \Sigma$ is required. However, a conventional liquid crystal compound having a relatively large value of $\Delta \Sigma$, such as 4-alkyl benzoic acid 4-cyanophenyl ester, 4-alkyl-4'-cyanobiphenyl, has a relatively low transition temperature between nematic phase and isotropic liquid phase (hereinafter referred to as "N-I point"), and renders the nematic temperature range relatively narrow. A compound having a relatively high N-I point and relatively large value of $\Delta \Sigma$, such as 4-alkyl-4"-cyanoterphenyl, 4-(trans-4'-alkyl-cyclohexyl)-4"-cyanobiphenyl, has a large value of elastic constant, thereby increasing the threshold voltage.

WO-A-8604081 discloses a liquid crystal monofluoro pyrimidine derivative. This liquid crystal monofluoro pyrimidine derivative has a relatively high threshold voltage.

The present invention seeks to provide pyrimidine derivatives from which liquid crystal compositions having a relatively high N-I point and having a relatively low threshold voltage can be created by admixture with one or more other nematic liquid crystal compounds.

According to the present invention, there is provided a pyrimidine derivative of the general formula:

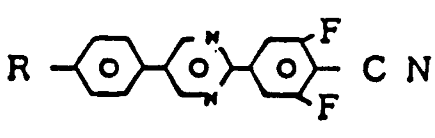

( 1 )

where R is a straight chain alkyl group or an alkoxy group having from 1 to 12 carbon atoms.

Pyrimidine derivatives of the general formula (1) are novel nematic liquid crystal compounds and have the features of a relatively high N-I point and of relatively large positive dielectric anisotropy Δ Σ . Accordingly, by using pyrimidine derivatives of general formula (1) in admixture with other liquid crystal compounds, it is possible to provide an improved liquid crystal display device having a relatively wide operating temperature range and a relatively low threshold voltage.

The pyrimidine derivatives of general formula (1) above may be prepared by the following reaction scheme:

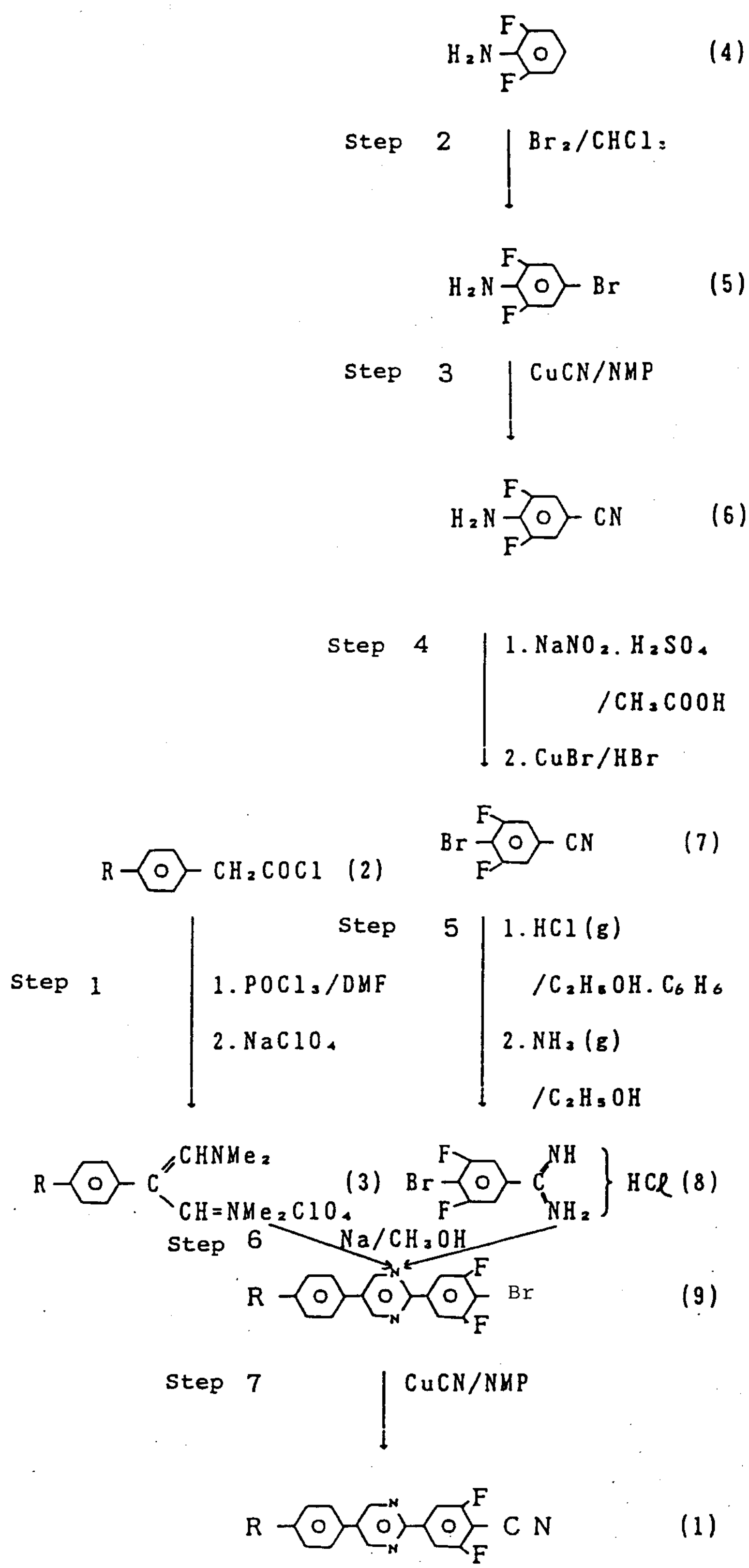


Step (1). Compound (2) is reacted with phosphorous oxychloride in dimethyl sulphoxide (PMF) and then sodium perchlorate to yield compound (3).
Step (2). Compound (4) is reacted with bromine in chloroform to yield compound (5).
Step (3). The compound (5) is reacted with copper (I) cyanide in N-methyl-2-pyrrolidone (NMP) to yield compound (6).

Step (4). The compound (6) is reacted with sodium nitride and sulphuric acid in acetic acid to yield the diazonium salt which is then reacted with copper (I) bromide in hydrobromic acid to yield compound (7).

Step (5). The compound (7) is reacted with dry hydrogen chloride gas in ethanol and benzene. The solvent is removed by distillation, and the resulting crystals reacted with dry ammonia gas in ethanol to yield compound (8).

Step (6). The compound (3) and the compound (8) are reacted with metallic sodium in methanol to yield compound (9).

Step (7). The compound (9) is reacted with copper (I) cyanide in N-methyl-2-pyrrolidone to yield the compound (1).

## EXAMPLE

Preparation of 2-(3',5'-difluoro-4'-cyanophenyl)-5-(4''-butylphenyl) pyrimidine

### STEP (1).

54 ml of phosphorous oxychloride was added dropwise to 230 ml of dimethylformamide, and then 42.1 g of 4-butylphenylacetylchloride was added dropwise. Then, after stirring for three hours at a temperature of 70 °C and with cooling, the dimethylformamide was removed by distillation. The residue was poured into ice water, and the excess phosphorous oxychloride was dissolved. Then an aqueous solution in which 26 g of sodium perchlorate had been dissolved, was added to the above residue and the mixture chilled. The crystallisation sample was filtered, and re-crystallisation repeated twice in ethanol to yield 58 g of 1-dimethylamino-3-dimethylimino-2-(4-butylphenyl)-propene-(1)-perchlorate.

### STEP (2).

100 g of 2, 6-difluoroaniline was dissolved in 180 ml of chloroform, and 140 g of bromine was added dropwise. After reflux for one hour, the resulting solution was poured into a solution of 10% potassium hydroxide. The mixture was extracted with chloroform and the organic layer was washed with a 10% aqueous solution of potassium hydroxide. After the chloroform had been removed by distillation, the residue was distillated under reduced pressure (bp70 to 80 °C/4mmHg), and re-crystallised from hexane to yield 127 g of 4-bromo-2, 6-difluoroaniline.

### STEP (3).

21 g of 4-bromo-2, 6-difluoroaniline, 11 g of copper (I) cyanide, and 70 ml of N-methyl-2-pyrrolidone were placed in a flask and maintained under reflux for three hours. The reaction mixture was poured into a solution formed by mixing 41 g of iron (III) chloride, 13 ml of concentrated hydrochloric acid, and 50 ml of water. The mixture was extracted with chloroform and washed with a 10% aqueous solution of potassium hydroxide, and then the chloroform was removed by distillation. The residue was distillated under reduced pressure (bp90 to 110 °C/4mmHg) and re-crystallised from a mixture of hexane and chloroform to yield 8.9 g of 4-cyano-2, 6-difluoro-aniline.

### STEP (4)

4 g of sodium nitride was added to 30 ml of concentrated sulphuric acid and chilled to below 10 °C. After that, 38 ml of acetic acid was added thereto. 8.9 g of 4-cyano-2, 6-difluoroaniline was added gradually in order to maintain the temperature of the solution between 20 °C and 25 °C. After stirring for one hour at the temperature of 20 °C to 25 °C, the reaction mixture was added dropwise to a solution of 10 g of copper (I) bromide dissolved in 30 ml of concentrated hydrobromic acid. After the dropwise addition had been completed, the reaction mixture was stirred for one and a half hours at room temperature. Then, water was added to the reaction mixture and the mixture extracted with chloroform and washed with water. The organic layer was removed by distillation, and the residue was re-crystallised from the mixture of methanol and acetone to yield 6.6 g of 2-bromo-5-cyano-1, 3-difluorobenzene.

**STEP (5).**

2 g of 2-bromo-5-cyano-1, 3-difluorobenzene was dissolved in a solution of 4 ml of ethanol and 15 ml of benzene, and chilled to a temperature below 0°C. Then dry hydrogen chloride gas was absorbed by this chilled solution for one hour. The solvent of the resulting solution was removed by distillation, and the crystalline residue was washed with ether. The resulting crystals were added to 4 ml of ethanol with stirring, the solution being chilled to a temperature of below 0°C. Then 16% ammonia/ethanol solution was added to the chilled solution, and stirred for one hour. After the reaction was completed, the solution was removed by distillation, and the residual crystals washed with ether to yield 2.1 g of 4-bromo-3, 5-difluorobenzamidine hydrochloride.

**STEP (6).**

2.1 g of 4-bromo-3, 5-difluorobenzamidine hydrochloride and 2.7 g of 1-dimethylamino-3-dimethylimino-2-(4-butylphenyl)-propene-(1)-perchlorate made in Step (1) were dissolved in 28 ml of methanol. A sodium methylate solution formed by 0.4 g of metallic sodium and 14 ml of methanol was added dropwise to the above resulting solution. The mixture was stirred overnight at room temperature, and maintained under reflux for one hour. The solution was chilled, water added thereto and the deposited crystals filtered. The crystals so obtained were re-crystallised from a solution of acetone and methanol to yield 2.1 g of 2-(3′, 5′-difluoro-4′-bromophenyl)-5-(4″-butylphenyl) pyrimidine.

**STEP (7).**

2.1 g of 2-(3′,5′-difluoro-4′-bromophenyl)-5-(4″-butylphenyl) pyrimidine, 0.8 g of copper (I) cyanide, and 6 ml of N-methyl-2-pyrrolidone were mixed in a flask, and the mixture maintained under reflux for one and a half hours. The resulting solution was poured into a mixture of 2.7 g of iron (III) chloride, 0.6 ml of concentrated hydrochloric acid, and 2.7 ml of water. The mixture was extracted with chloroform, and washed with water and 10% aqueous potassium hydroxide, and then the chloroform was removed by distillation. The residue was re-crystallised from a mixture of acetone and methanol to yield 0.5 g of 2-(3′,5′-difluoro-4′-cyanophenyl)-5-(4″-butylphenyl) pyrimidine. The resulting compound showed nematic liquid crystal properties, a melting point of 101°C and an N-I point of 144°C.

The following compounds can be prepared in a similar manner:

2-(3′,5′-difluoro-4′-cyanophenyl)-5-(4″-methylphenyl) pyrimidine;
2-(3′,5′-difluoro-4′-cyanophenyl)-5-(4″-ethylphenyl) pyrimidine;
C → N 171°C, N → I 163°C
(where C represents the crystal phase, N represents the nematic phase, and I represents the isotropic phase).
2-(3′,5′-difluoro-4′-cyanophenyl)-5-(4''-propylphenyl) pyrimidine
C → N 136°C, N → I 155.5°C
2-(3′,5′-difluoro-4′-cyanophenyl)-5-(4″-pentylphenyl) pyrimidine
C → N 114.5°C, N → I 145.5°C
2-(3′,5′-difluoro-4′-cyanophenyl)-5-(4″-hexylphenyl) pyrimidine
2-(3′,5′-difluoro-4′-cyanophenyl)-5-(4″-heptylphenyl) pyrimidine
C → N 108°C, N → I 142°C
2-(3′,5′-difluoro-4′-cyanophenyl)-5-(4″-octylphenyl) pyrimidine
2-(3′,5′-difluoro-4′-cyanophenyl)-5-(4″-nonylphenyl) pyrimidine
2-(3′,5′-difluoro-4′-cyanophenyl)-5-(4″-decylphenyl) pyrimidine
2-(3′,5′-difluoro-4′-cyanophenyl)-5-(4″-undecylphenyl) pyrimidine
2-(3′,5′-difluoro-4′-cyanophenyl)-5-(4″-dodecylphenyl) pyrimidine
C → N 121°C, N → I 140°C
2-(3′,5′-difluoro-4′-cyanophenyl)-5-(4″-methoxyphenyl) pyrimidine
2-(3′,5′-difluoro-4′-cyanophenyl)-5-(4″-ethoxyphenyl) pyrimidine
C → N 170°C, N → I 165°C
2-(3′,5′-difluoro-4′-cyanophenyl)-5-(4″-propyloxyphenyl) pyrimidine
C → N 135°C, N → I 193°C
2-(3′,5′-difluoro-4′-cyanophenyl)-5-(4″-butyloxyphenyl) pyrimidine
2-(3′,5′-difluoro-4′-cyanophenyl)-5-(4″-pentyloxyphenyl) pyrimidine,
C → N 106.5°C, N → I 179°C

2-(3′,5′-difluoro-4′-cyanophenyl)-5-(4″-hexyloxyphenyl) pyrimidine
C → N 132.5 °C, N → I 175.5 °C
2-(3′,5′-difluoro-4′-cyanophenyl)-5-(4″-heptyloxyphenyl) pyrimidine
2-(3′,5′-difluoro-4′-cyanophenyl)-5-(4″-octyloxyphenyl) pyrimidine
2-(3′,5′-difluoro-4′-cyanophenyl)-5-(4″-nonyloxyphenyl) pyrimidine
C → N 124 °C, N → I 168 °C
2-(3′,5′-difluoro-4′-cyanophenyl)-5-(4″-decyloxyphenyl) pyrimidine
2-(3′,5′-difluoro-4′-cyanophenyl)-5-(4″-undecyloxyphenyl) pyrimidine
2-(3′,5′-difluoro-4′-cyanophenyl)-5-(4″-dodecyloxyphenyl) pyrimidine
C → N 143 °C, N → I 165 °C

Next, reference is made to comparative tests of properties of 4-pentyl-4″-cyanoterphenyl which is used in order to improve the N-I point generally and the compound of the above Example, namely, 2-(3′,5′-difluoro-4′-cyanophenyl)-5-(4″-butylphenyl) pyrimidine in a mixed base liquid crystal material ZLI-1565 (made by Merck Co. Ltd., N-I point 89.3 °C).

First of all, the following compositions A and B were prepared.

[Composition A]

ZLI-1565 90% part by weight

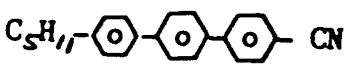
10% part by weight

[Composition B]

ZLI-1565 90% part by weight

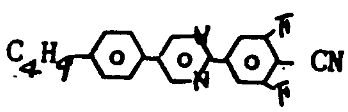
10% part by weight

Each composition A and B was inserted into a 7 micron thickness twisted nematic cell. Then, the voltage transmittance properties (Figure 2) under alternating static driving at a temperature of 25 °C was measured. The results are shown in the following Table.

TABLE

| Composition | N-I point | $V_{10}$ | $\alpha$ | $\beta$ |
|---|---|---|---|---|
| A | 105.5 | 2.344 | 1.289 | 1.412 |
| B | 92.9 | 1.794 | 1.277 | 1.375 |

In the above Table, $V_{10}$ is a voltage value at 10% transmittance, and V is a measured value from the measuring direction of $\theta = 90°$. Further, $\alpha$ and $\beta$ are factors showing the viewing angle properties and threshold properties, respectively, and $\alpha$ and $\beta$ are defined as:

$\alpha = \theta 90° \, V_{50} / \theta 50° \, V_{50}$

$\beta = \theta 90° \, V_{10} / \theta 90° \, V_{90}$

As mentioned above, by mixing a pyrimidine derivative according to the present invention with a general liquid crystal composition, the N-I point goes up, the value of the threshold voltage reduces markedly and the electro-optical properties (values of $\alpha$ and $\beta$ ) are improved. In view of this, the present invention is particularly useful as a basic component of a liquid crystal composition for super twisted nematic type liquid crystal display devices widely used at present.

In the drawings, Figure 1 is an illustration showing the infra-red spectra of 2-(3′,5′-difluoro-4′-cyanophenyl)-5-(4″-butylphenyl) pyrimidine according to the present invention, and Figures 2 (a) and (b) are

illustrations showing the voltage transmittance properties of a general twisted nematic cell and the measuring direction $\theta$ thereof. The curve $\theta 50°$ shows the voltage transmittance properties from a measuring direction $\theta = 50°$, and curve $\theta 90°$ shows the same from a measuring direction $\theta = 90°$. $V_{10}$, $V_{50}$ and $V_{90}$ show the value of voltages at the time of 10%, 50% and 90% transmittance respectively.

## Claims

1. A pyrimidine derivative of the general formula:

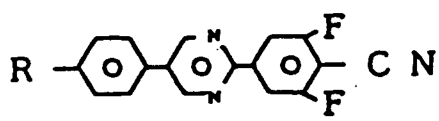

where R is a straight chain alkyl group or an alkoxy group having from 1 to 12 carbon atoms.

2. 2-(3′,5′-difluoro-4′-cyanophenyl)-5-(4″-methylphenyl) pyrimidine, or
2-(3′,5′-difluoro-4′-cyanophenyl)-5-(4″-ethylphenyl) pyrimidine, or
2-(3′,5′-difluoro-4′-cyanophenyl)-5-(4″-propylphenyl) pyrimidine, or
2-(3′,5′-difluoro-4′-cyanophenyl)-5-(4″-butylphenyl) pyrimidine, or
2-(3′,5′-difluoro-4′-cyanophenyl)-5-(4″-pentylphenyl) pyrimidine, or
2-(3′,5′-difluoro-4′-cyanophenyl)-5-(4″-hexylphenyl) pyrimidine, or
2-(3′,5′-difluoro-4′-cyanophenyl)-5-(4″-heptylphenyl) pyrimidine, or
2-(3′,5′-difluoro-4′-cyanophenyl)-5-(4″-octylphenyl) pyrimidine, or
2-(3′,5′-difluoro-4′-cyanophenyl)-5-(4″-nonylphenyl) pyrimidine, or
2-(3′,5′-difluoro-4′-cyanophenyl)-5-(4″-decylphenyl) pyrimidine, or
2-(3′,5′-difluoro-4′-cyanophenyl)-5-(4″-undecylphenyl) pyrimidine, or
2-(3′,5′-difluoro-4′-cyanophenyl)-5-(4″-dodecylphenyl) pyrimidine.

3. 2-(3′,5′-difluoro-4′-cyanophenyl)-5-(4″-methoxyphenyl) pyrimidine, or
2-(3′,5′-difluoro-4′-cyanophenyl)-5-(4″-ethoxyphenyl) pyrimidine, or
2-(3′,5′-difluoro-4′-cyanophenyl)-5-(4″-propyloxyphenyl) pyrimidine, or
2-(3′,5′-difluoro-4′-cyanophenyl)-5-(4″-butyloxyphenyl) pyrimidine, or
2-(3′,5′-difluoro-4′-cyanophenyl)-5-(4″-pentyloxyphenyl) pyrimidine, or
2-(3′,5′-difluoro-4′-cyanophenyl)-5-(4″-hexyloxyphenyl) pyrimidine, or
2-(3′,5′-difluoro-4′-cyanophenyl)-5-(4″-heptyloxyphenyl) pyrimidine, or
2-(3′,5′-difluoro-4′-cyanophenyl)-5-(4″-octyloxyphenyl) pyrimidine, or
2-(3′,5′-difluoro-4′-cyanophenyl)-5-(4″-nonyloxyphenyl) pyrimidine, or
2-(3′,5′-difluoro-4′-cyanophenyl)-5-(4″-decyloxyphenyl) pyrimidine, or
2-(3′,5′-difluoro-4′-cyanophenyl)-5-(4″-undecyloxyphenyl) pyrimidine, or
2-(3′,5′-difluoro-4′-cyanophenyl)-5-(4″-dodecyloxyphenyl) pyrimidine.

## Patentansprüche

1. Ein Pyrimidinderivat der allgemeinen Formel:

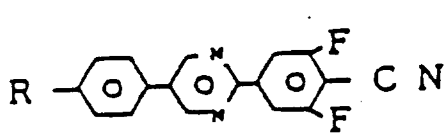

in der R eine geradkettige Alkylgruppe oder eine Alkoxygruppe mit 1 bis 12 Kohlenstoffatomen ist.

2. 2-(3',5' Difluor-4'-cyanphenyl)-5-(4''-methylphenyl)pyrimidin oder
2-(3',5'Difluor-4'-cyanphenyl)-5-(4''-ethylphenyl)pyrimidin oder
2-(3',5'Difluor-4'-cyanphenyl)-5-(4''-propylphenyl)pyrimidin oder
2-(3',5'-Difluor-4'-cyanphenyl)-5-(4''-butylphenyl)pyrimidin oder

2-(3',5'-Difluor-4'-cyanphenyl)-5-(4''-pentylphenyl)pyrimidin oder
2-(3',5'Difluor-4'-cyanphenyl)-5-(4''-hexylphenyl)pyrimidin oder
2-(3',5'-Difluor-4'-cyanphenyl)-5-(4''-heptylphenyl)pyrimidin oder
2-(3',5'-Difluor-4'-cyanphenyl)-5-(4''-octylphenyl)pyrimidin oder
2-(3',5'-Difluor-4'-cyanphenyl)-5-(4''-nonylphenyl)pyrimidin oder
2-(3',5'-Difluor-4'-cyanphenyl)-5-(4''-decylphenyl)pyrimidin oder
2-(3',5'-Difluor-4'-cyanphenyl)-5-(4''-undecylphenyl)pyrimidin oder
2-(3',5'-Difluor-4'-cyanphenyl)-5-(4''-dodecylphenyl)pyrimidin.

**3.** 2-(3',5'-Difluor-4'-cyanphenyl)-5-(4''-methoxyphenyl)pyrimidin oder
2-(3',5'-Difluor-4'-cyanphenyl)-5-(4''-ethoxyphenyl)pyrimidin oder
2-(3',5'-Difluor-4'-cyanphenyl)-5-(4''-propyloxyphenyl)pyrimidin oder
2-(3',5'-Difluor-4'-cyanphenyl)-5-(4''-butyloxyphenyl)pyrimidin oder
2-(3',5'-Difluor-4'-cyanphenyl)-5-(4''-pentyloxyphenyl)pyrimidin oder
2-(3',5'-Difluor-4'-cyanphenyl)-5-(4''-hexyloxyphenyl)pyrimidin oder
2-(3',5'-Difluor-4'-cyanphenyl)-5-(4''-heptyloxyphenyl)pyrimidin oder
2-(3',5'-Difluor-4'-cyanphenyl)-5-(4''-octyloxyphenyl)pyrimidin oder
2-(3',5'-Difluor-4'-cyanphenyl)-5-(4''-nonyloxyphenyl)pyrimidin oder
2-(3',5'-Difluor-4'-cyanphenyl)-5-(4''-decyloxyphenyl)pyrimidin oder
2-(3',5'-Difluor-4'-cyanphenyl)-5-(4''-undecyloxyphenyl)pyrimidin oder
2-(3',5'-Difluor-4'-cyanphenyl)-5-(4''-dodecyloxyphenyl)pyrimidin.

## Revendications

**1.** Dérivé de la pyrimidine de la formule générale :

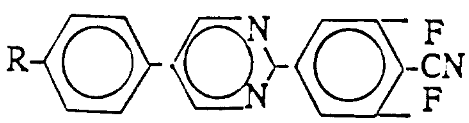

dans laquelle R est un groupe alkyle ou un groupe alcoxy à chaîne droite ayant de 1 à 12 atomes de carbone.

**2.** 2-(3',5'-difluoro-4'-cyanophényl)-5-(4''-méthylphényl)pyrimidine, ou
2-(3'5'-difluoro-4'-cyanophényl)-5-(4''-éthylphényl)pyrimidine, ou
2-(3',5'-difluoro-4'-cyanophényl)-5-(4''-propylphényl)pyrimidine, ou
2-(3',5'-difluoro-4'-cyanophényl)-5-(4''-butylphényl)pyrimidine, ou
2-(3',5'-difluoro-4'-cyanophényl)-5-(4''-pentylphényl)pyrimidine, ou
2-(3',5'-difluoro-4'-cyanophényl)-5-(4''-hexylphényl)pyrimidine, ou
2-(3',5'-difluoro-4'-cyanophényl)-5-(4''-heptylphényl)pyrimidine, ou
2-(3',5'-difluoro-4'-cyanophényl)-5-(4''-octylphényl)pyrimidine, ou
2-(3',5'-difluoro-4'-cyanophényl)-5-(4''-nonylphényl)pyrimidine, ou
2-(3',5'-difluoro-4'-cyanophényl)-5-(4''-décylphényl)pyrimidine, ou
2-(3',5'-difluoro-4'-cyanophényl)-5-(4''-undécylphényl)pyrimidine, ou
2-(3',5'-difluoro-4'-cyanophényl-5-(4''-dodécylphényl)pyrimidine.

**3.** 2-(3',5'-difluoror-4'-cyanophényl)-5-(4''-méthoxyphényl)pyrimidine, ou
2-(3',5'-difluoro-4'-cyanophényl)-5-(4''-éthoxyphényl)pyrimidine, ou
2-(3',5'-difluoro-4'-cyanophényl)-5-(4''-propyloxyphényl)pyrimidine, ou
2-(3',5'-difluoro-4'-cyanophényl)-5-(4''-butyloxyphényl)pyrimidine, ou
2-(3',5'-difluoro-4'-cyanophényl)-5-(4''-pentyloxyphényl)pyrimidine, ou
2-(3',5'-difluoro-4'-cyanophényl)-5-(4''-hexyloxyphényl)pyrimidine, ou
2-(3',5'-difluoro-4'-cyanophényl)-5-(4''-heptyloxyphényl)pyrimidine, ou
2-(3',5'-difluoro-4'-cyanophényl)-5-(4''-octyloxyphényl)pyrimidine, ou
2-(3',5'-difluoro-4'-cyanophényl)-5-(4''-nonyloxyphényl)pyrimidine, ou
2-(3',5'-difluoro-4'-cyanophényl)-5-(4''-décycloxyphényl)pyrimidine, ou

2-(3',5'-difluoro-4'-cyanophényl)-5-(4''-undécyloxyphényl)pyrimidine, ou
2-(3',5'-difluoro-4'-cyanophényl)-5-(4''-dodécyloxyphènyl)pyrimidine.

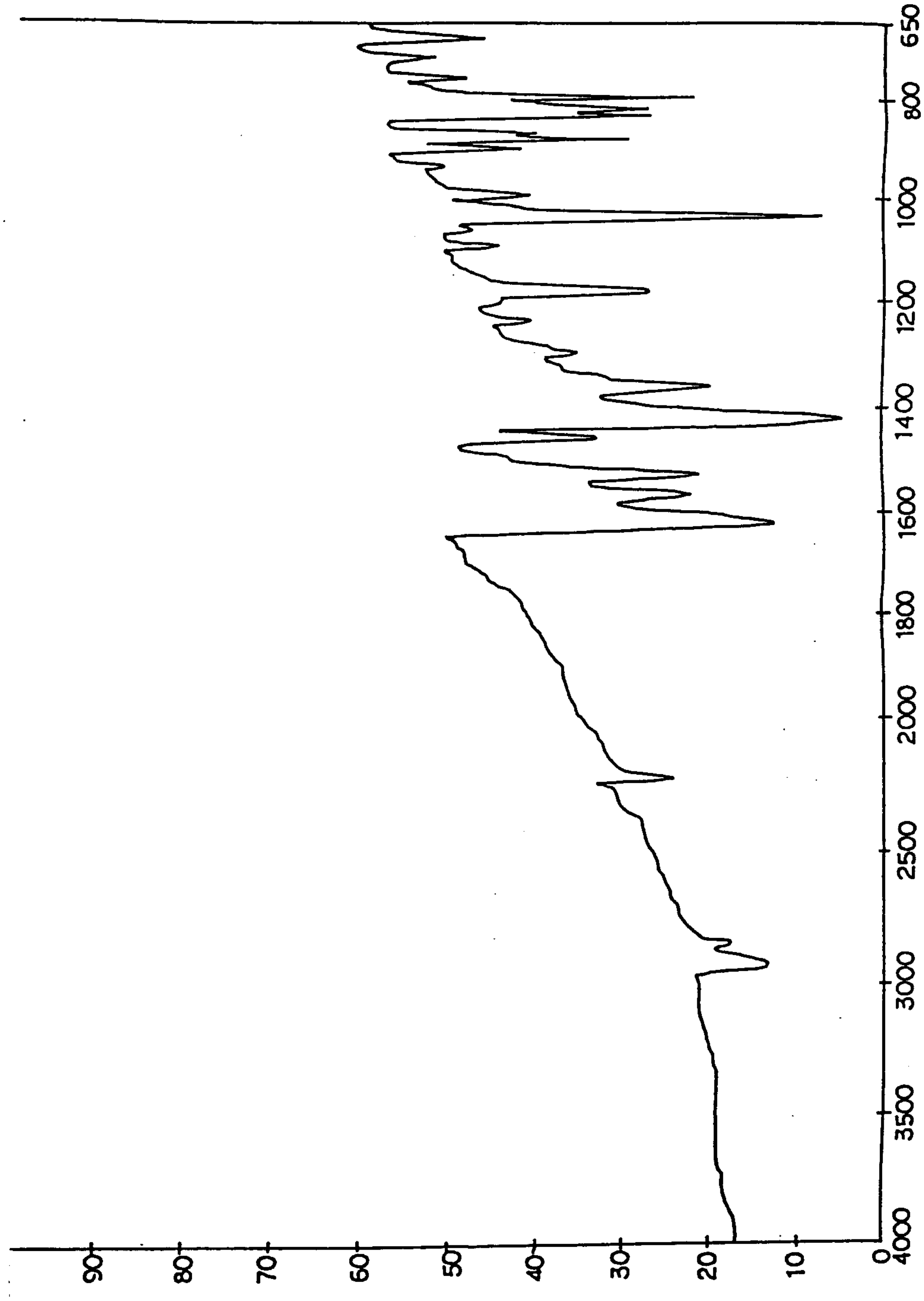
90
80
70
60
50
40
30
20
10
0
4000
3500
3000
2500
2000
1800
1600
1400
1200
1000
800
650
CM-1

FIG 1

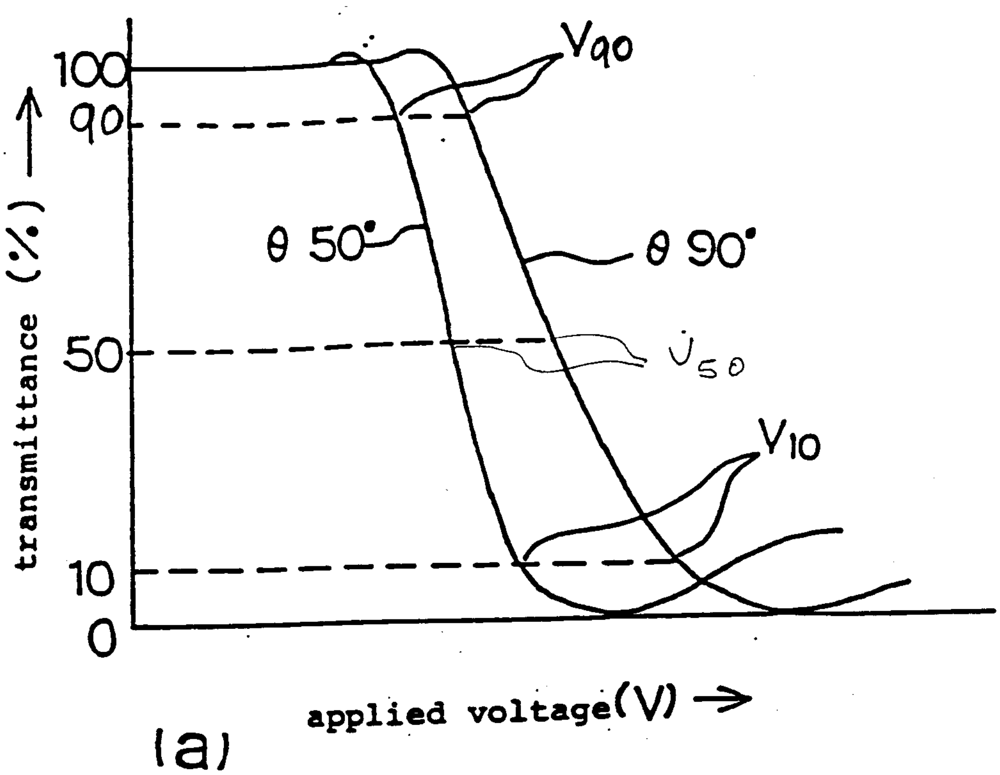
100
90
50
10
0
transmittance (%) →
applied voltage(V) →
V90
θ 50°
θ 90°
V50
V10

(a)

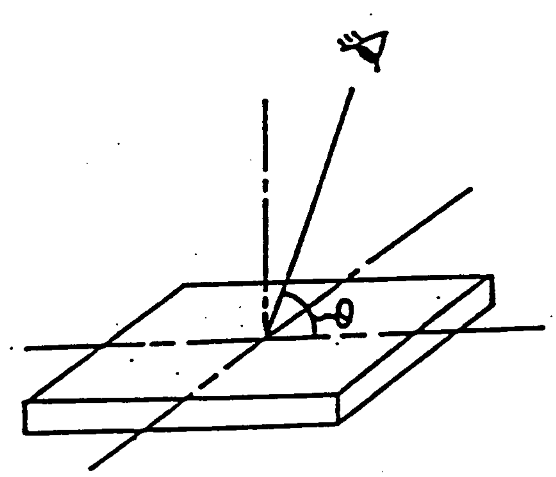
θ

(b)

F I G 2